(19) Europäisches Patentamt
European Patent Office
Office européen des brevets

(11) EP 2 052 719 B1

(12) **EUROPEAN PATENT SPECIFICATION**

(45) Date of publication and mention of the grant of the patent:
**16.01.2013 Bulletin 2013/03**

(51) Int Cl.:
***A61K 31/085*** (2006.01) ***A61K 31/216*** (2006.01)
***A61K 31/265*** (2006.01) ***A61P 25/16*** (2006.01)
***A61P 25/28*** (2006.01)

(21) Application number: **08253402.5**

(22) Date of filing: **21.10.2008**

(54) **Curative drug for neurodegenerative diseases**

Heilendes Arzneimittel für neurodegenerative Erkrankungen

Médicament curatif pour maladies neurodégénératives

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.10.2007 JP 2007275790**

(43) Date of publication of application:
**29.04.2009 Bulletin 2009/18**

(73) Proprietor: **NIPPON HYPOX LABORATORIES INCORPORATED**
**Tokyo 192-0354 (JP)**

(72) Inventors:
- **Sugiyama, Satoru**
  **Nagoya-shi Aichi 464-0833 (JP)**
- **Miki, Tokutaro**
  **Tokyo, 192-0354 (JP)**
- **Nishikawa, Hiroshi**
  **Shizuoka-shi, Shizuoka,420-0882 (JP)**

(74) Representative: **Thomas, Simon**
**Urquhart-Dykes & Lord LLP**
**3rd Floor**
**33 Glasshouse Street**
**London W1B 5DG (GB)**

(56) References cited:
**DE-A1- 2 300 543** **JP-A- 2002 241 366**
**JP-A- 2004 352 661** **US-A- 5 416 242**

- **HINO T ET AL: "HTHQ (1-O-hexyl-2,3,5-trimethylhydroquinone), an anti-lipid-peroxidative compound: its chemical and biochemical characterizations" BIOCHIMICA ET BIOPHYSICA ACTA - GENERAL SUBJECTS, ELSEVIER SCIENCE PUBLISHERS, NL, vol. 1425, no. 1, 16 September 1998 (1998-09-16), pages 47-60, XP004276189 ISSN: 0304-4165**
- **SHIGEMATSU S. et al: "Standard Pathology", 15 March 1997 (1997-03-15), IGAKU SHOIN LTD, Tokyo pages 669-679,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 052 719 B1

## Description

This invention relates to a curative drug for neurodegenerative diseases, and particularly to preventive and curative drugs for neurodegenerative diseases such as Alzheimer's disease and Parkinson's disease.

## Background and Prior Art

There is a pressing need to develop preventive and curative drugs for neurodegenerative diseases such as Alzheimer's disease and Parkinson's disease, which increase in critical frequency with advancing age.

In the neurodegenerative diseases, different body sites on the body are affected, but diseases such as nerve cell degeneration and cell mortality are found anywhere on these sites. The studies on the cause and mechanism of the diseases have continued until now, but they remain to be explained. As curative drugs for those diseases, there have been used a cholinesterase inhibitor for Alzheimer's disease, and drugs for inhibiting dopamine degradation such as L-dopa preparation, dopamine-releasing promoter or COMT inhibitor for Parkinson's disease. However, these drugs cannot inhibit impairment of neuronal cells and are ineffective at stopping progression of symptoms.

Meanwhile, involvement of oxidative stress has been singled out as a possible cause of these diseases. The undermentioned Non-patent Literature 1 discloses the possibility of decreasing the oxidative stress, which results from various causes such as beta-amyloid having vitamin E accumulated in plenty in the brain of an Alzheimer's disease sufferer, ischemia reperfusion injury or aluminum accumulation, to cause a cerebroprotective action, thereby to slow the progression of Alzheimer's disease. The undermentioned Non-patent Literature 2 reports an effect of retarding progression to profound dementia, which can be brought about by administering to a moderate Alzheimer's disease sufferer high-dose vitamin E and selegiline which is an antioxidant agent used as an antiparkinsonism drug in a clinical experiment.
Non-patent Literature 1: Koichi Abe et al. "Vitamin" Vol. 74, No. 3, ppl 13-119(2000)
Non-patent Literature 2: M. Sano et al., N Engl J Med, 336, 1216-1222(1997)

Although the administration of the antioxidant agent shows a likelihood and effect of slowing the progression of condition of the neurodegenerating disease to some extent, prevention of disease and significant improvements in disease presentation cannot be expected. Consequently, it is awkward to say that sufficient effect can be acquired. Thus, there has been a call for development of new drugs for the neurodegenerative disease, which has a beneficial effect capable of inhibiting nerve cell disorder due to the oxidative stress, thereby to stop progression of symptoms.

In the meanwhile, there is a compound formulated by the following general Formula 1, which specifically has a highly antioxidative effect and biocompatibility among hydroquinone derivatives having the antioxidative effect. In specific, this compound has more antioxidative activity than an antioxidant agent such as vitamin E and a potent effect of suppressing the production of nitric oxide (NO), which leads to increase in oxidative stress. An invention with respect to the compound and compositions using the compound, such as an oxidation inhibitor, therapeutic agent for refractory inflammatory diseases, cancerogenesis inhibitor, and therapeutic composition for arteriosclerosis is disclosed.

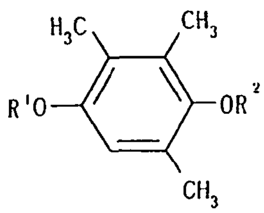

..Formula 1

(wherein, $R^1$ represents an alkyl group with a carbon number of 4 to 8, and $R^2$ represents a hydrogen atom, alkylcarbonyl group with a carbon number of 2 to 6 or alkoxycarbonyl group with a carbon number of 2 to 6.)

As one example, Patent Literature 1 enumerated below discloses an antioxidant agent consisting of the aforesaid compound as an active constituent. This citation further discloses that the antioxidant agent has more antioxidant properties than butylhydroxytoluene which is a typical food antioxidant agent and has lower toxicity to a biologic body.

Patent Literature 2 drafted by the inventors of this invention discloses the aforesaid compound has the antioxidative effect to block production of NO, thus to have efficacy as the therapeutic agent for refractory inflammatory diseases such as arthritis rheumatoides and nonspecific inflammatory bowel disease. Patent Literature 3 discloses a cancerogenesis inhibitor containing the aforesaid compound as an active constituent. Patent Literature 4 drafted by the inventors of this invention discloses therapeutic composition for liver disorder containing the aforesaid compound as an active constituent. Further, Patent Literature 5 drafted by the inventors of this invention discloses that the aforesaid compound has an effect of preventing cholesterol oxidation, which is exerted *in vivo* as a therapeutic agent for arteriosclerosis, and can be used safely.

It is reported in Non-patent Literature 3 that the aforesaid compound having a carbon number $R^1$ of 4 to 8 in an alkyl group typically has high antioxidative properties; in particular, the compound having the carbon number of 6 shows the highest antioxidative properties. Non-patent Literature 4 discloses that 2,3,5-trimethyl-hydroquinone-1-hexylether among the hydroquinone derivatives expressed in the general Formula 1 above has antioxidative properties twice as strong as vitamin E and a NO-production inhibitory activity 500 times as strong as vitamin E.

However, none of Patent Literatures 1 to 5 and Non-patent Literatures 1 to 4 disclose consideration of the behavior of the aforesaid compound on nerve cells and the inhibitory effect of the aforesaid compound on nerve cell disorder and practical exemplification of the aforesaid compound to be used as a curative drug for neurodegenerative diseases.

Patent Literature 6 discloses that 2-isopropylhydroquinone, which is a kind of hydroquinone derivatives, but not pertinent to the aforesaid compound as expressed in the general Formula 1, has a function of promoting biosynthesis of a nerve growth factor. However, this does not describe functions other than the biosynthesis promoting function, particularly the effect of suppressing oxidative stress. There is no description about which derivative among the hydroquinone derivatives is the most effective as a curative drug for nerve disease from the standpoint of a pharmacologic activity and biocompatibility.

Patent Literature 1: Japanese Unexamined Pat. Appln. Publication HEI 05-301836

Patent Literature 2: Japanese Unexamined Pat. Appln. Publication No. 2004-352661

Patent Literature 3: Japanese Unexamined Pat. Appln. Publication HEI 06-100441

Patent Literature 4: Japanese Unexamined Pat. Appln. Publication HEI 08-67627

Patent Literature 5: Japanese Unexamined Pat. Appln. Publication No. 2002-241366

Patent Literature 6: Japanese Examined Pat. Appln. Publication HEI 7-110812

Non-patent Literature 3: Y. Nihro et al., Chem Pharm Bull, 42, 576-579 (1994)

Non-patent Literature 4: Wei Liu et al., J Pharm Pharmacol, 54, 383-389 (2002)

JP 2004-352661 discloses compounds for the treatment of intractable inflammatory disease.

DE 23 00543 discloses phenol ethers as anti-parkinson drugs.

From the aforesaid standpoint, the inventors of this invention were made repeated keen studies on the effectiveness of hydroquinone derivative for neurodegenerative diseases, consequently to reveal that the hydroquinone derivative expressed in the aforesaid general Formula 1 has potent specific effect of protecting nerve cells from oxidative stress. Thus, the present invention was achieved on the basis of the revealed efficacy as a curative drug for neurodegenerative diseases.

## Summary of the Invention

According to the invention, there is provided a compound for use in the curative treatment of a neurodegenerative disease, wherein the compound is represented by Formula 1 or a cyclodextrin inclusion compound thereof

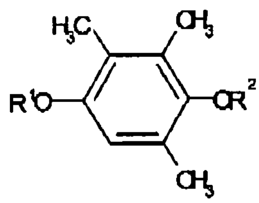

..Formula 1

wherein,

$R^1$ represents an alkyl group with a carbon number of 4 to 8, and
$R^2$ represents a hydrogen atom, an alkylcarbonyl group with a carbon number of 2 to 6 or an alkoxycarbonyl group with a carbon number of 2 to 6.

Further according to the invention, there is provided a pharmaceutical composition for use in the curative treatment of a neurodegenerative disease comprising as an active constituent a compound according to the invention.

Even further according to the invention, there is provided the use of a compound according to the invention in the preparation of a medicament for use in the curative treatment of a neurodegenerative disease.

It is an object of the present invention to provide a curative drug for neurodegenerative diseases, containing, as an active constituent, a compound represented by Formula 1 or a cyclodextrin inclusion compound thereof:

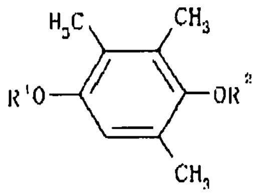

..Formula 1

wherein, $R^1$ represents an alkyl group with a carbon number of 4 to 8, and $R^2$ represents a hydrogen atom, alkylcarbonyl group with a carbon number of 2 to 6 or alkoxycarbonyl group with a carbon number of 2 to 6.

Another object of the invention is to provide the curative drug for neurodegenerative diseases as described above, in which the compound represented by the aforementioned Formula 1 is 2,3,5-trimethyl-hydroquinone-1-hexylether or 2,3,5-trimethyl-hydroquinone-1-hexylether 4-acetate.

The hydroquinone derivative according to the invention, which consists of the compound represented by the aforementioned Formula 1 or cyclodextrin inclusion compound has an effect of strongly protecting nerve cells from oxidative stress and can be used safely. Thus, the composition containing the hydroquinone derivative as an active constituent according to the invention can be effectively used as the curative drug for neurodegenerative diseases.

In particular, the compound consisting of 2,3,5-trimethyl-hydroquinone-1-hexylether or 2,3,5-trimethyl-hydroquinone-1-hexylether 4-acetate according to the invention is excellent in terms of a pharmacologic activity and biocompatibility, and therefore, can be used more effectively.

Further advantages and specific details of the invention will be set forth hereinafter in conjunction with the following detailed description of a presently preferred embodiment.

The invention will be better understood and objects other than those set forth above will become apparent when consideration is given to the following detailed description thereof.

## Detailed Description of the Invention

In the compound contained in the curative drug for neurodegenerative diseases according to the invention, which is represented by the aforementioned Formula 1, the alkyl group with a carbon number of 4 to 8 indicated by $R^1$ may optionally assume a straight chain, branched chain or cyclic structure. For instance, there may be various types of butyl group, pentyl group, hexyl group, heptyl group, octyl group, cyclobutyl group, cyclopentyl group, cyclohexyl group, cycloheptyl group, and cyclooctyl group. From the aspect of a pharmacologic activity and biocompatibility, the compound having a straight-chain structure with a carbon number of 4 to 7 is preferable, and the hexyl group is particularly preferable.

The alkylcarbonyl group with a carbon number of 2 to 6 in $R^2$ may assume a straight chain structure or a branched chain structure. For instance, acetyl group, propionyl group, butyryl group, and isobutyryl group can be enumerated. Also, the alkoxycarbonyl group with a carbon number of 2 to 6 in $R^2$ may be a straight chain structure or a branched chain structure. For instance, methoxycarbonyl group, ethoxycarbonyl group, propoxycarbonyl group and isopropoxycarbonyl group can be enumerated.

As a preferable compound as represented by Formula 1 above in terms of a pharmacologic activity, 2,3,5-trimethyl-hydroquinone-1-butyl ether, 2,3,5-trimethyl-hydroquinone-1-hexylether, and 2,3,5-trimethyl-hydroquinone-1-hexylether 4-acetate can be enumerated.

As one example, the compound as represented by Formula 1 and the cyclodextrin inclusion compound thereof can be produced by a method described in the aforesaid Patent Literature 5.

The curative drug for neurodegenerative diseases according to the invention contains, as an active constituent, a compound represented by Formula 1 or a cyclodextrin inclusion compound thereof, and is prepared by adding pharmaceutically approved additives such as a pharmaceutical carrier and excipients. The curative drug of the invention may assume many and varied forms as conventionally applied for medicinal purposes, for example, in a orally-bioavailable form capable of being readily absorbed from the digestive tract such as of a tablet, granulated powder, capsules or liquid medication, a parenteral form such as a transdermal absorption drag as prepared in a injectable liquid or suppository form, or a soluble solid or liquid form which can be dissolved with a suitable solvent in use in consideration for circulation and storage stability. Furthermore, application of a technique for a blood-brain transferring mechanism is especially

useful for the curative drug for neurodegenerative diseases of the invention.

A dose of the curative drug for neurodegenerative diseases of the invention depends on various factors such as an intended curative effect, administration method, age and weight of a subject. Therefore, the dose of the curative drug is not definitively determined, but a daily dosage for parenteral administration is generally about 0.01 to 100 mg, preferably 0.05 to 10 mg, per kilogram of body weight. For oral administration, a daily dosage is about 0.1 to 300 mg, preferably 0.5 to 100 mg. The prescribed amount of curative drug may be orally administered daily at a time or in two to five divided doses.

**Examples**

The medical experiment carried out according to the present invention will be described hereinafter in detail, but the invention should not be understood as being limited to the experiments.

[Experimental Example: Cytoprotective properties with cultured cells] Of the hydroquinone derivatives expressed in the aforesaid Formula 1, 2,3,5-trimethyl-hydroquinone-1-hexylether (Compound 1) was examined in comparison with vitamin E with respect to the activity of inhibiting toxicity of lipid peroxide to nerve cells. PC 12 cells as a cell culture model of for human neuroblastoma-derived SH-SY5Y and rat pheochromocytoma cells were cultivated by the law of the art. A culture media was prepared in effect by adding FCS (fetal calf serum) to Dulbecco's Eagle medium (DMEM) and used further with the addition of $NaHCO_3$ (3.7g/L), penicillin G (100U/mL) and Streptomycin (100mg/ml). A cultivation was performed by seeding $6 \times 10^4$ SH-SY5Y cells and $4 \times 10^4$ PC12 cells in a CO2 incubator at 37°C using a microplate with 24 macroscopic pores. After 48 hours, the aforesaid Compound 1 or vitamin E was added thereto. Then after about 30 minutes, 70 μM oflinoleic acid hydroperoxide were added thereto for the SH-SY5Y cells and 35 μM of the same were added for PC 12 cells. After cultivation for about 20 hours, the number of surviving cells was measured.

The measurement of the surviving cells was made by a Methylene blue dye uptake method. To be more specific, the cells were rinsed twice with PBS (phosphate buffered saline free from Mg and Ca), and then, fixated with 10% formalin for 5 minutes. Further, the cells were rinsed three times with distilled water, stained with 0.05% methylene blue solution for 60 minutes, and rinsed three times with distilled water. Then, 20 minutes after adding 0.33N HCl thereto, the absorbance of the cells was measured at 665 nm. The survival rate and protection rate of the cells were calculated on the basis of the measured result by the following equation.

$$\text{Cell survival rate (\%)} = [\text{absorbance of treated cells with testing additives}] / [\text{absorbance of non-treated cells}] \times 100$$

$$\text{Protection rate (\%)} = \{1 - (100 - [\text{survival rate of treated cells with testing additives}]) / (100 - [\text{survival rate of cells with no additives}])\} \times 100$$

The experimental results on SH-SY5Y cells are shown in Table 1 below, and the experimental results on PC12 cells are shown in Table 2 below.

[Table 1]

| Effect on cell disorder due to linoleic acid hydroperoxide (LA-OOH) on SH-SY5Y cells | | | | |
|---|---|---|---|---|
| Test Substance | Dosage (μM) | LA-OOH (μM) | Survival Rate (%) | Protection Rate (%) |
| Vitamin E | 0 | 0 | 100.00 | - |
| | 0 | 70 | 80.97 | 0.0 |
| | 0.5 | 70 | 83.72 | 14.5 |
| | 1.0 | 70 | 79.77 | -6.3 |
| | 5.0 | 70 | 79.78 | -6.2 |
| | 10.0 | 70 | 81.78 | 4.3 |

(continued)

| Effect on cell disorder due to linoleic acid hydroperoxide (LA-OOH) on SH-SY5Y cells | | | | |
|---|---|---|---|---|
| Compound 1 | 0 | 0 | 100.00 | - |
| | 0 | 70 | 80.52 | 0.0 |
| | 0.5 | 70 | 83.41 | 14.8 |
| | 1.0 | 70 | 84.83 | 22.1 |
| | 5.0 | 70 | 87.97 | 38.3 |
| | 10.0 | 70 | 87.04 | 33.5 |
| Compound 1: 2,3,5-trimethyl-hydroquinone-1-hexylether | | | | |

[Table 2]

| Effect on cell disorder due to linoleic acid hydroperoxide (LA-OOH) on PC12 cells | | | | |
|---|---|---|---|---|
| Test Substance | Dosage (μM) | LA-OOH (μM) | Survival Rate (%) | Protection Rate (%) |
| Vitamin E | 0 | 0 | 100.00 | - |
| | 0 | 35 | 32.61 | 0.0 |
| | 0.1 | 35 | 37.16 | 6.8 |
| | 0.5 | 35 | 35.88 | 4.9 |
| | 1.0 | 35 | 35.85 | 4.8 |
| | 5.0 | 35 | 42.57 | 14.8 |
| Compound 1 | 0 | 0 | 100.00 | - |
| | 0 | 35 | 30.76 | 0.0 |
| | 0.1 | 35 | 45.68 | 21.5 |
| | 0.5 | 35 | 86.57 | 80.6 |
| | 1.0 | 35 | 91.96 | 88.4 |
| | 5.0 | 35 | 92.31 | 88.9 |
| Compound 1: 2,3,5-trimethyl-hydroquinone-1-hexylether | | | | |

It is apparent from Table 1 and Table 2 that Compound 1 exerts an effect of validly restraining cyopathy due to linoleic acid hydroperoxide on both the SH-SY5Y cells and PC 12 cells in comparison with vitamin E and advantageously has lower toxicity.

The protection rate of Compound 1 added to the cells is far higher than a value expected merely from a cell protecting effect from oxidative stress due to an antioxidative effect, i.e. an expected value predictable from the fact that the antioxidative effect of Compound 1 is double that of vitamin E. Particularly, the survival rate and protection rate of PC12 cells according to Compound 1 both are up to around 90%, which is notably higher than vitamin E showing a sirvival rate up to about 42% and a protection rate of around 15% for the PC12 cells. The cause is still not known, but it is assumed that the hydroquinone derivative has possibly behaviors contributing to protection and survival of nerve cells, other than the antioxidative effect. That is, as one of those effective behaviors, it has been reported that the hydroquinone derivative is considered to have a promotive effect for stimulating biosynthesis of nerve growth factor. The survival rate and protection rate of the cells may possibly be increased due to a synergy of the antioxidative effect and the cellular protection effect, promoting growth and survival of the nerve cells. The exceptional effects of the Compound 1 of the invention are clearly beneficial as the curative drug for neurodegenerative diseases.

From the experimental results as described above, it is evident that the compound according to the present invention is significantly effective for protecting neuronal cells from oxidative stress due to lipid peroxide.

While the invention has been explained by reference to particular embodiments thereof, and while these embodiments have been described in considerable detail, the invention is not limited to the representative medicinal substance and medicine manufacture as described. Accordingly, the scope of the invention is to be determined by the following claims.

## Claims

1. A compound for use in the curative treatment of a neurodegenerative disease, wherein the compound is represented by Formula 1 or cyclodextrin inclusion compound thereof

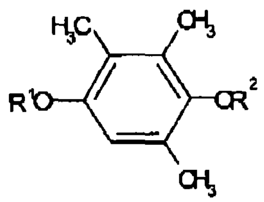

..Formula 1

wherein,

$R^1$ represents an alkyl group with a carbon number of 4 to 8, and
$R^2$ represents a hydrogen atom, an alkylcarbonyl group with a carbon number of 2 to 6 or an alkoxycarbonyl group with a carbon number of 2 to 6.

2. The compound for use in the curative treatment of a neurodegenerative disease according to claim 1, wherein the compound represented by Formula 1 is 2,3,5-trimethyl-hydroquinone-1-hexylether or 2,3,5-trimethyl-hydroquinone-1-hexylether 4-acetate.

3. The compound for use in the curative treatment of a neurodegenerative disease according to claim 1 or claim 2, wherein the neurodegenerative disease is Alzheimer's disease or Parkinson's disease.

4. A pharmaceutical composition for use in the curative treatment of a neurodegenerative disease comprising as an active constituent a compound of Formula 1 according to any one of claims 1 to 3.

5. The composition for use in the curative treatment of a neurodegenerative disease according to claim 4, comprising a pharmaceutical carrier and/or excipient.

6. Use of a compound represented by Formula 1 or a cyclodextrin inclusion compound thereof

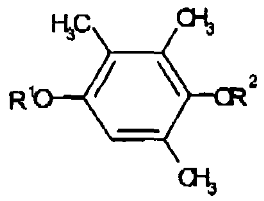

..Formula 1

wherein,

$R^1$ represents an alkyl group with a carbon number of 4 to 8, and
$R^2$ represents a hydrogen atom, an alkylcarbonyl group with a carbon number of 2 to 6 or an alkoxycarbonyl group with a carbon number of 2 to 6, in the preparation of a medicament for use in the curative treatment of a neurodegenerative disease.

7. Use according to claim 6, wherein the compound represented by Formula 1 is 2,3,5-trimethyl-hydroquinone-1-hexylether or 2,3,5-trimethyl-hydroquinone-1-hexylether 4-acetate.

8. Use according to claim 6 or claim 7, wherein the neurodegenerative disease is Alzheimer's disease or Parkinson's disease.

## Patentansprüche

1. Verbindung zur Verwendung bei der Heilbehandlung einer neurodegenerativen Erkrankung, wobei die Verbindung durch die Formel 1 dargestellt wird, oder eine Cyclodextrin-Einschlussverbindung davon:

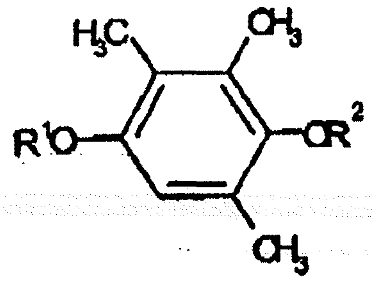

Formel 1

wobei

$R^1$ für eine Alkylgruppe mit einer Kohlenstoffanzahl von 4 bis 8 steht und
$R^2$ für ein Wasserstoffatom, eine Alkylcarbonylgruppe mit einer Kohlenstoffanzahl von 2 bis 6 oder eine Alkoxycarbonylgruppe mit einer Kohlenstoffanzahl von 2 bis 6 steht.

2. Verbindung zur Verwendung bei der Heilbehandlung einer neurodegenerativen Erkrankung nach Anspruch 1, wobei die durch die Formel 1 dargestellte Verbindung 2,3,5-Trimethylhydrochinon-1-hexylether oder 2,3,5-Trimethylhydrochinon-1-hexylether-4-acetat ist.

3. Verbindung zur Verwendung bei der Heilbehandlung einer neurodegenerativen Erkrankung nach Anspruch 1 oder 2, wobei die neurodegenerative Erkrankung Alzheimer-Krankheit oder Parkinson-Krankheit ist.

4. Pharmazeutische Zusammensetzung zur Verwendung bei der Heilbehandlung einer neurodegenerativen Erkrankung, die eine Verbindung der Formel 1 nach einem der Ansprüche 1 bis 3 als einen Wirkbestandteil umfasst.

5. Zusammensetzung zur Verwendung bei der Heilbehandlung einer neurodegenerativen Erkrankung nach Anspruch 4, die einen pharmazeutischen Trägerstoff und/oder Hilfsstoff umfasst.

6. Verwendung einer Verbindung, die durch die Formel 1 dargestellt wird, oder eine Cyclodextrin-Einschlussverbindung davon:

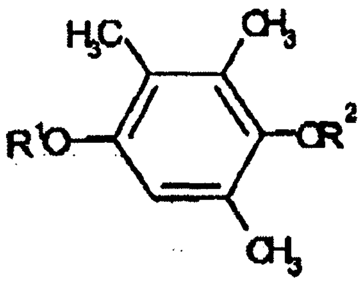

Formel 1

wobei

$R^1$ für eine Alkylgruppe mit einer Kohlenstoffanzahl von 4 bis 8 steht und

$R^2$ für ein Wasserstoffatom, eine Alkylcarbonylgruppe mit einer Kohlenstoffanzahl von 2 bis 6 oder eine Alkoxycarbonylgruppe mit einer Kohlenstoffanzahl von 2 bis 6 steht, bei der Herstellung eines Arzneimittels zur Verwendung bei der Heilbehandlung einer neurodegenerativen Erkrankung.

**7.** Verwendung nach Anspruch 6, wobei die durch die Formel 1 dargestellte Verbindung 2,3,5-Trimethylhydrochinon-1-hexylether oder 2,3,5-Trimethylhydrochinon-1-hexylether-4-acetat ist.

**8.** Verwendung nach Anspruch 6 oder 7, wobei die neurodegenerative Erkrankung Alzheimer-Krankheit oder Parkinson-Krankheit ist.

## Revendications

**1.** Composé destiné à être utilisé dans le traitement curatif d'une maladie neurodégénérative, le composé étant représenté par la formule 1, ou composé d'inclusion de celui-ci avec la cyclodextrine

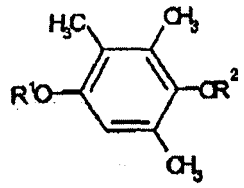

Formule 1

où

$R^1$ représente un groupe alkyle ayant un nombre d'atomes de carbone de 4 à 8 et
$R^2$ représente un atome d'hydrogène, un groupe alkylcarbonyle ayant un nombre d'atomes de carbone de 2 à 6 ou un groupe alcoxycarbonyle ayant un nombre d'atomes de carbone de 2 à 6.

**2.** Composé destiné à être utilisé dans le traitement curatif d'une maladie neurodégénérative selon la revendication 1, le composé représenté par la formule 1 étant le 1-éther d'hexyle et de 2,3,5-triméthylhydroquinone ou le 4-acétate du 1-éther d'hexyle et de 2,3,5-triméthylhydroquinone.

**3.** Composé destiné à être utilisé dans le traitement curatif d'une maladie neurodégénérative selon la revendication 1 ou la revendication 2, la maladie neurodégénérative étant la maladie d'Alzheimer ou la maladie de Parkinson.

**4.** Composition pharmaceutique destinée à être utilisée dans le traitement curatif d'une maladie neurodégénérative comprenant comme constituant actif un composé de formule 1 selon l'une quelconque des revendications 1 à 3.

**5.** Composition destinée à être utilisée dans le traitement curatif d'une maladie neurodégénérative selon la revendication 4, comprenant un véhicule et/ou excipient pharmaceutique.

**6.** Utilisation d'un composé représenté par la formule 1 ou d'un composé d'inclusion de celui-ci avec la cyclodextrine

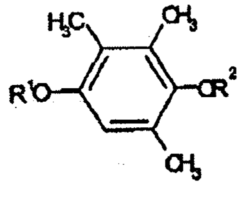

Formule 1

où

$R^1$ représente un groupe alkyle ayant un nombre d'atomes de carbone de 4 à 8 et
$R^2$ représente un atome d'hydrogène, un groupe alkylcarbonyle ayant un nombre d'atomes de carbone de 2 à 6 ou un groupe alcoxycarbonyle ayant un nombre d'atomes de carbone de 2 à 6, dans la préparation d'un médicament destiné à être utilisé dans le traitement curatif d'une maladie neurodégénérative.

**7.** Utilisation selon la revendication 6, le composé représenté par la formule 1 étant le 1-éther d'hexyle et de 2,3,5-triméthylhydroquinone ou le 4-acétate du 1-éther d'hexyle et de 2,3,5-triméthylhydroquinone.

**8.** Utilisation selon la revendication 6 ou la revendication 7, la maladie neurodégénérative étant la maladie d'Alzheimer ou la maladie de Parkinson.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI05301836 B
- JP 2004352661 A
- JP HEI06100441 B
- JP HEI0867627 B
- JP 2002241366 A
- JP HEI7110812 B
- DE 2300543


### Non-patent literature cited in the description


- **KOICHI ABE et al.** *Vitamin,* 2000, vol. 74 (3), 13-119
- **M. SANO et al.** *N Engl J Med,* 1997, vol. 336, 1216-1222
- **Y. NIHRO et al.** *Chem Pharm Bull,* 1994, vol. 42, 576-579
- **WEI LIU et al.** *J Pharm Pharmacol,* 2002, vol. 54, 383-389